(19) Europäisches Patentamt / European Patent Office / Office européen des brevets

(11) **EP 0 922 449 B1**

(12) **EUROPEAN PATENT SPECIFICATION**

(45) Date of publication and mention
of the grant of the patent:
**03.01.2007 Bulletin 2007/01**

(51) Int Cl.:
*A61K 9/16* (2006.01)      *A61K 9/20* (2006.01)
*A61K 9/50* (2006.01)

(21) Application number: **98120608.9**

(22) Date of filing: **30.10.1998**

(54) **Use of an enzymatically converted starch derivative as an encapsulating agent**

Verwendung eines enzymatisch behandelten Stärkederivates als Einkapselungsmaterial

Utilisation d'un dérivé d'amidon traité par enzymes en tant qu'agent d'encapsulage

(84) Designated Contracting States:
**AT BE CH CY DE DK ES FI FR GB GR IE IT LI LU MC NL PT SE**

(30) Priority: **31.10.1997 US 962282**
**23.10.1998 US 178087**

(43) Date of publication of application:
**16.06.1999 Bulletin 1999/24**

(60) Divisional application:
**03020600.7 / 1 371 363**

(73) Proprietor: **National Starch and Chemical Investment Holding Corporation Wilmington, Delaware 19803-7663 (US)**

(72) Inventors:
• **Blue, Emily Keller**
  **Indianapolis,**
  **Indiana 46268 (US)**
• **Chiu, Chung-Wai**
  **Westfield, New Jersey 07090 (US)**
• **Hussain, Zahera**
  **Hyde Park,**
  **New York 12538 (US)**
• **Jeffcoat, Roger**
  **Bridgewater,**
  **New Jersey 08807 (US)**
• **Shah, Himanshu**
  **Branchburg,**
  **New Jersey 08876 (US)**
• **Turbiano, Paul**
  **Somerville,**
  **New Jersey 08876 (US)**
• **Boyd, Dennis**
  **Neshanic Station,**
  **New Jersey 08853 (US)**

(74) Representative: **Held, Stephan et al**
  **Meissner, Bolte & Partner GbR**
  **Postfach 86 03 29**
  **81630 München (DE)**

(56) References cited:
**EP-A- 0 332 027**          **US-A- 3 455 838**
**US-A- 4 035 235**          **US-A- 5 354 559**
**US-A- 5 468 286**

• **PATENT ABSTRACTS OF JAPAN vol. 17, no. 496 (C-1108), 8 September 1993 (1993-09-08) & JP 05 132401 A (MATSUTANI KAGAKU KOGYO KK), 28 May 1993 (1993-05-28) & DATABASE WPI Week 199326 Derwent Publications Ltd., London, GB; AN 208813**
• **PATENT ABSTRACTS OF JAPAN vol. 1995, no. 05, 30 June 1995 (1995-06-30) & JP 07 053324 A (NISSHIN OIL MILLS LTD), 28 February 1995 (1995-02-28) & DATABASE WPI Week 199517 Derwent Publications Ltd., London, GB; AN 128207**

**Description**

The present invention relates to a modified starch which is prepared by enzymatic hydrolysis of a starch molecule after the preparation of a starch derivative containing a hydrophobic group or both a hydrophobic and a hydrophilic group. Such modified starch is useful as an encapsulating agent, particularly in systems where high load and retention of the active ingredient, low surface oil exposure, and excellent oxidation resistance is desired. The encapsulating agent is useful in numerous applications, including a tablet.

U.S. Patent Nos. 4,977,252 and 5,185,176 issued to Chiu disclose starch derivatives containing a hydrophobic or both a hydrophobic and a hydrophilic group which have been enzymatically degraded by exo-enzymes. These modified starches are useful as emulsifiers.

A variety of chemical compositions are conventionally used as encapsulating agents in, *inter alia*, the food, cosmetic, paint, pharmaceutical, personal care, household, and polymer industries. Typical compositions which conventionally function as encapsulating agents include gum arabic, dextrins, low viscosity modified starches, arabinogalactan, gum acacia, casein, gelatin, carboxymethyl cellulose, tragacanth, karaya, sodium alginate, tannin, and celluloses.

These typical compositions however do not consistently provide high active agent loading and retention, low surface oil and excellent oxidation resistance. In general, powders prepared with conventional encapsulating agents do not contain a high level of active agents. When loaded with oil levels of higher than 15-20%, such conventional encapsulated powders lose a considerable amount of the oil during the drying process, have much of the oil exposed on the surface of the powder, and/or generally have poor oxidation resistance

U.S. Patent No. 3,971,852 issued to Brenner, et al. discloses a method for encapsulating oils in particles of a solid water-sensitive, preferably water-soluble, protective matrix that isolates the oils until they are released for use by exposure of the particles to moisture. The matrix-forming encapsulation materials include mixtures of polysaccharides and polyhydroxy compounds that can form aqueous emulsions with the oil. Although the patent claims efficient encapsulation of up to 80% by volume and surface oil not substantially above 5%, with a relatively high loading, the known process fails to provide efficient encapsulation oil recovery with excessive oil loss during drying and extractable oils as high as 10-24% when the encapsulated oil content exceeds 60% by weight. Furthermore, it has not been shown that these matrices provide good oxidation resistance.

U.S. Patent No. 5,087,461 issued to Levine, et al. discloses a spray dried composition encapsulated in an extruded glassy matrix composed of a chemically modified starch having a dextrose equivalent no greater than 2, a maltodextrin, a corn syrup solid or polydextrose, and a mono- or di-saccharide. However, these encapsulated products are unable to achieve high loading and are susceptible to oxidation.

EP Patent Application No. 550 067 A1 discloses a method for encapsulating oils in a water-sensitive cellular solid matrix by drying an aqueous emulsion containing the oil to be encapsulated, a non-crosslinked lipophilically modified starch that undergoes crosslinking during drying, and a polyhydroxy compound that forms with the polysaccharide material a continuous aqueous phase in which the oil is dispersible as a discontinuous phase. This method of encapsulation is unacceptable for foods and requires the incorporation of a silicore-based material which is difficult to process using conventional methods. Furthermore, it has not been shown that these matrices provide good oxidation resistance.

However, none of the encapsulating agents known in the art provide a high load of at least 40% while maintaining a retention of at least 90% of the active ingredient with less than 3% surface oil exposure, and excellent oxidation resistance for a wide variety of encapsulating agents.

Compressed tablets are also well-known, particularly in the pharmaceutical industry. Known methods of tabletting include direct compression and wet or dry granulation followed by compression. Tablet formulations characteristically should be free flowing, cohesive and lubricating. Sometimes, it is desired to encapsulate a component of the tablet and gelatin is considered as a standard encapsulating agent in many industries.

However, consumers may desire products which do not contain gelatin for a variety of reasons including dietary to meet strict Kosher, Halal or vegetarian standards. Many consumers also want to avoid bovine products because of the current scare over Bovine Spongiform Encephalopathy (Mad Cow Disease). Further, gelatin is an expensive excipient and its replacement is desirable to reduce the cost of the product.

It is known in the art that certain starches are excellent encapsulating agents. However, as starches are generally used as disintegrants, impeding compression and hardness of the tablet, starch encapsulating agents are not generally used in significant quantities in tablets. Hardness is necessary in a tablet as it provides resistance to chipping, abrasion, and breakage under conditions of storage, transportation, and handling prior to consumer consumption.

Other encapsulating agents generally do not allow for good compressibility while providing high load and retention of the active agent and oxidative resistance.

Surprisingly, it has now been discovered that the present invention which uses a modified starch, prepared by enzymatically converting a starch after the preparation of a starch derivative containing a hydrophobic group or a hydrophobic and a hydrophilic group, as an encapsulating agent, may consistently allow for such high load and retention of a variety of active ingredients and low oil exposure while providing excellent oxidation resistance. It has also been discovered that the encapsulating agent comprising such modified starch allows similar compressibility characteristics and resultant hardness to gelatin in a tablet formulation.

The present invention is directed to a modified starch which is prepared by enzymatic hydrolysis of a starch molecule after the preparation of a starch derivative containing a hydrophobic group or both a hydrophobic and a hydrophilic group. Such modified starch is useful as an encapsulating agent, particularly in systems where high load and retention of the active ingredient, low surface oil exposure, and excellent oxidation resistance is desired.

The present invention is also directed to a tablet, particularly a pharmaceutical dosage form, which contains the encapsulating agent. The encapsulating agent allows for good compressibility and hardness of the tablet. Further, it may allow for high load and retention of a variety of active agents as well as oxidative resistance.

An object of the present invention is to provide an enzymatically converted, starch derivative containing a hydrophobic group or a hydrophobic and a hydrophilic group which is useful as an encapsulating agent and a method of producing such starch.

Another object of the present invention is to provide a β-amylase or glucoamylase hydrolyzed, hydrophobically derivatized starch.

Still another object of the present invention is to provide a β-amylase or glucoamylase hydrolyzed, alkenyl succinic starch.

Yet another object of the present invention is to provide products containing an enzymatically converted, starch derivative containing a hydrophobic group or a hydrophobic and a hydrophilic group as an encapsulating agent and a method of preparing such products.

A further object of the present invention is to provide products containing a β-amylase or glucoamylase hydrolyzed, hydrophobically derivatized starch as an encapsulating agent.

A still further object of the present invention is to provide products containing a β-amylase or glucoamylase hydrolyzed, alkenyl succinic starch as an encapsulating agent.

A yet further object of the present invention is to provide a blend of a starch derivative containing a hydrophobic group or a hydrophobic and a hydrophilic group and a mono-, di-, or oligo-saccharide or a maltodextrin which is useful as an encapsulating agent.

An additional object is to provide a tablet, such as a pharmaceutical tablet, which contains such encapsulating agents.

The present invention is directed to a modified starch which is prepared by enzymatic hydrolysis of a starch molecule after the preparation of a starch derivative containing a hydrophobic group or both a hydrophobic and a hydrophilic group. Such modified starch is useful as an encapsulating agent, particularly in systems where high load and retention of the active ingredient, low surface oil exposure, and excellent oxidation resistance is desired. Further, such encapsulating agents can be processed at high solids during the encapsulation process.

The present invention is also directed to a tablet, particularly a pharmaceutical dosage form, which contains the encapsulating agent. The encapsulating agent allows for good compressibility and hardness of the tablet. Further, it may allow for high load and retention of a variety of active agents as well as oxidative resistance.

All starches and flours are suitable for use herein and may be derived from any native source. A native starch or flour, as used herein, is one as it is found in nature, including those developed by plant breeding, and bioengineered starches. Typical sources for the starches and flours are cereals, tubers, roots, legumes and fruits. The native source can be corn, pea, potato, sweet potato, banana, barley, wheat, rice, sago, amaranth, tapioca, arrowroot, canna, sorghum, and waxy or high amylose varieties thereof. As used herein, the term "waxy" is intended to include a starch or flour containing at least about 95% by weight amylopectin and the term "high amylose" is intended to include a starch or flour containing at least about 45% by weight amylose. In particular, corn, waxy maize, tapioca, potato, and rice are useful in the instant invention.

Also included as useful base starch materials are the conversion products derived from any of the above starches including fluidity or thin-boiling starches prepared by oxidation, α-amylase conversion, mild acid hydrolysis or heat dextrinization, and derivatized starch such as ethers and esters.

A particularly useful starch base is a gelatinized starch, that is a precooked, non-granular starch, and also may be a fluidity starch converted by mild acid degradation or heat dextrinization methods that are well known in the art. For example, see Rutenberg, "Starch and Its Modifications," *Handbook of Water-Soluble Gums and Resins*, Davidson, Editor, McGraw-Hill, Inc., New York, N.Y. 1980, pp.22-36. A combination of one or more of these conversion techniques may be used. The conversion is typically carried out before treatment with a hydrophobic or a hydrophobic/hydrophilic reagent and before the enzyme treatment. If desired, the starch base may be converted by treatment with an α-amylase enzyme to produce a fluidity starch in the manner disclosed in U.S. Patent No. 4,035,235. Where a high viscosity system is desired, it is not necessary to convert the base starch.

The starch may be derivatized by treatment with any reagent or combination of reagents which contributes encapsulation properties to the starch. The reagent must contain a hydrophobic moiety and may contain a hydrophilic moiety The hydrophobic moiety should be an alkyl or alkenyl group which contains at least five carbon atoms, or an aralkyl or aralkenyl group which contains at least six carbon atoms, particularly up to about twenty-four carbon atoms. The hydrophilic moiety may be contributed by the reagent or the starch's own hydroxyl groups may serve as the hydrophilic moiety and the reagent may contribute only the hydrophobic moiety.

Any process for derivatizing starch which yields the desired blend of hydrophobic or hydrophobic and hydrophilic functions on the starch molecule and thereby yields stable encapsulation properties may be used to prepare the modified starch of the present invention. Suitable derivatives and methods for producing them are known in the art and disclosed in U.S. Patent No. 4,626,288.

In a particularly useful embodiment, the starch is derivatized by reaction with an alkenyl cyclic dicarboxylic acid anhydride by the method disclosed in U.S. Patent Nos. 2,613,206 and 2,661,349 or propylene oxide, more particularly by reaction with octenylsuccinic anhydride.

Where a low viscosity is desirable, a particularly useful embodiment is an octenyl succinic half ester derivative of an amylopectin containing starch, such as waxy maize, which has been converted to a water fluidity (WF) of up to about 60. Water fluidity is an empirical test of viscosity measured on a scale of 0-90 wherein fluidity is the reciprocal of viscosity. Water fluidity of starches is typically measured using a Thomas Rotational Shear-type Viscometer (commercially available from Arthur A. Thomas CO., Philadelphia, PA), standardized at 30°C with a standard oil having a viscosity of 24.73 cps, which oil requires $23.12 \pm 0.05$ sec for 100 revolutions. Accurate and reproducible measurements of water fluidity are obtained by determining the time which elapses for 100 revolutions at different solids levels depending on the starch's degree of conversion: as conversion increases, the viscosity decreases. In a particularly useful embodiment, the converted starch is treated with from about 0.1% to about 30% for food products, and at least about 0 1% for other products, of octenyl succinic anhydride. In the alternative, a hydroxypropyl octenyl succinic derivative may be used.

For other products, any degree of substitution or level of conversion that results in the desired viscosity and encapsulation properties may be employed. For example, U.S. Patent No. 4,035,235 disclosed a suitable embodiment comprising a method for producing a hydrophobic derivative of starch to be used as an alternative to gum arabic in encapsulating water insoluble substances, such as volatile flavoring oils and perfumes.

After derivatizing the starch, it is further enzymatically hydrolyzed by at least one enzyme capable of cleaving the 1,4-linkages of the starch molecule from the non-reducing ends to produce mono- and/or di-saccharides to provide high oxidation resistance, particularly those with less than about three glucose units, while maintaining substantially high molecular weight portions of the starch base to provide encapsulating properties. The enzymes useful in the present invention thus include, but are not limited to, β-amylase, glucoamylase, maltogenase, pullulanase, exo-alpha-1,4-glucosidase, exo-1,4-alpha-D-glucan maltotetrahydrolase, and exo-1,4-alpha-D glucan maltohexahydrolase, particularly β-amylase, glucoamylase.

The enzymatic hydrolysis of the starch base is carried out using techniques known in the art. The amount of enzyme used is dependent upon the enzyme, i.e., type, source and activity, and base material used as well as the amount of hydrolysis desired. Typically, the enzyme is used in an amount of from about 0.01 to about 1.0%, particularly from about 0.01 to 0.3%, by weight of the starch.

The optimum parameters for enzyme activity will vary depending upon tne enzyme used. The rate of enzyme degradation depends upon factors known in the art, including the type of enzyme used, enzyme concentration, substrate concentration, pH, temperature, the presence or absence of inhibitors, and the degree and type of modification. These parameters may be adjusted to optimize the digestion rate of the starch base.

The starch may be gelatinized before enzyme hydrolysis. The gelatinization process unfolds the starch molecules from the granular structure, thereby permitting the enzyme to more easily and uniformly degrade the starch molecules.

Generally the enzyme treatment is carried out in an aqueous or buffered slurry at a starch solids level of about 10 to about 40%, depending upon the base starch being treated. A solids level of from about 15 to 35% is particularly useful, from about 18 to 25% more particularly useful, in the instant invention. In the alternative, the process may utilize an enzyme immobilized on a solid support.

Typically, enzyme digestion is carried out at the highest solids content feasible without reducing reaction rates in order to facilitate any desired subsequent drying of the starch composition. Reaction rates may be reduced by high solids content as agitation becomes difficult or ineffective and the starch dispersion becomes more difficult to handle.

The pH and temperature of the slurry should be adjusted to provide effective enzyme hydrolysis. These parameters are dependent upon the enzyme to be used and are known in the art. In general, a temperature of about 22 to about 65°C is used, particularly from about 50 to about 62°C. In general, the pH is adjusted to about 3.5 to about 7.5, particularly from about 4.0 to about 6.0, using techniques known in the art.

The enzyme reaction is continued until a dextrose equivalent of from 20 to 50, has been achieved, or until the desired end point (i.e., sufficient degradation to provide the desired functionality for the particular application) has been reached. The end point may be determined by a change in viscosity, by reducing sugar content (such as measured by dextrose equivalents), or by any other method known in the art for measuring the level of enzyme degradation of the starch molecule. In general, the enzyme reaction will take from about 0.1 to about 24 hours, particularly about 0.5 to about 4 hours. The time of the reaction is dependent upon the type of starch and enzyme used, the amount of enzyme used, and the reaction parameters of solids percent, pH, and temperature.

The enzyme degradation is then terminated by any technique known in the art such as acid or base deactivation, heat deactivation, ion exchange, and solvent extraction. For example, acid deactivation may be accomplished by adjusting the pH to lower than 2.0 for at least 30 minutes or heat deactivation may be accomplished by raising the temperature to about 85 to about 95°C and maintaining it at that temperature for at least about 10 minutes to fully deactivate the enzyme. Heat deactivation is not suitable if a granular product is desired as the heat necessary to deactivate the enzyme will generally also gelatinize the starch.

The resultant solution is typically adjusted to the desired pH according to its intended end use. In general, the pH is adjusted to from about 5.0 to about 7.5, particularly from about 6.0 to about 7.0, using techniques known in the art. The modified starch is then typically dried using methods known in the art, particularly spray drying. However, the modified starch may also be used as a liquid concentrate.

The resulting starch is characterized by a relatively low viscosity, moderately high dextrose equivalent, neutral taste, and by its unique functionality as an encapsulating agent.

The viscosity of the resultant starch should be less than about 30 seconds, particularly from about 8 to about 25 seconds, more particularly from about 8 to about 15 seconds as measured by the funnel method. Viscosity is an important parameter in contributing to efficient encapsulation.

To measure the viscosity of the starch by the funnel method, the starch dispersion to be tested is adjusted to 19% or 25% (w/w) measured by refractometer. The temperature of the dispersion is controlled at 22°C. A total of 100 ml of the starch dispersion is measured into a graduated cylinder. It is then poured into a calibrated funnel while using a finger to close the orifice. A small amount is allowed to flow into the graduate to remove any trapped air and the balance is poured back into the funnel. The graduated cylinder is then inverted over the funnel so that the contents draw (flow) into the funnel while the sample is running. Using a timer, the time required for the 100 ml sample to flow through the apex of the funnel is recorded.

The glass portion of the funnel is a standard 58°, thick-wall, resistance glass funnel whose top diameter is about 9 to about 10 cm with the inside diameter of the stem being about 0.381 cm. The glass stem of the funnel is cut to an approximate length of 2.86 cm from the apex, carefully fire-polished, and refitted with a long stainless steel tip with is about 5.08 cm long with an outside diameter of about 0.9525 cm. The interior diameter of the steel tip is about 0.5952 cm at the upper end where is attached to the glass stem and about 0.4445 cm at the outflow end with the restriction in the width occurring at about 2.54 cm from the ends. The steel tip is attached to the glass funnel by means of a Teflon tube. The funnel is calibrated so as to allow 100 ml of water to go through in six seconds using the above procedure.

The resultant starch should have a dextrose equivalent of from 20 to 50. When glucoamylase is used to hydrolyze the derivatized starch, the DE is particularly from about 30 to about 50. When β-amylase is used, the DE is particularly from about 20 to about 50, more particularly from about 25 to about 38. Dextrose equivalent (DE) is defined as the reducing power of the hydrolyzate. Each starch molecule has one reducing end: therefore DE is inversely related to molecular weight. The DE of anhydrous D-glucose is defined as 100 and the DE of unhydrolyzed starch is virtually zero.

In the alternative, the derivatized starch may be blended with sugars, for example mono- di- or oligo-saccharides or maltodextrins, instead of producing the sugars *in situ*. The mono- di, and oligo-saccharides include all saccharides of up to about 10 glucose units, particularly those of up to about 3 glucose units, such as glucose, fructose, galactose, maltose, isomaltose, sucrose, lactose, raffinose, stachyose, fructosylsucrose, and maltooligosaccharides, particularly glucose, fructose, and maltose. The maltodextrins include those with a dextrose equivalent of from about 2 to about 50, particularly from about 5 to about 15.

The resultant starch/sugar blend should have a relatively high percent of sugars measured as glucose, at least about 20 and up to about 80%, particularly from about 40 to about 60%, sugar by weight.

The resultant starches (or starch/sugar blends), when used as encapsulating agents, have the advantages of achieving and maintaining consistently high load levels, low oil exposure, and excellent oxidation resistance.

The active agents may be encapsulated using the modified starches of the present invention and techniques known in the art, including but not limited to spray drying, extrusion, spray chilling, and fluid bed coating. For example, the starch may be dispersed in water, the active agent may be added and emulsified, and the emulsion may then be spray dried to form the encapsulated product.

When a starch /sugar blend is used, spray drying efficiencies may be decreased due to the high viscosity of the system and greater dryer deposits, particularly when mono-saccharides are used. Spray efficiencies may be increased by methods known in the art, such as using high drying towers, lightly oiling the chamber walls, or using preconditioned air in which the moisture has been substantially removed.

The active agent may be any substance which will not react with the starch system, including but not limited to oils, fats, flavors, colors, fragrances, vitamins, and pharmaceuticals. In particular, the modified starch of the present invention is useful for encapsulating oil-based active agents such as flavor oils and vitamins. These oils may be volatile or non-volatile and are generally characterized by being water immiscible but dispersible (emulsifiable) in water in the presence of an encapsulating agent.

The encapsulated product prepared with the present encapsulating agents consistently achieve and maintain a relatively high load level of the active agent. The load level of the active agent realized may be greater than 40%, particularly greater than 50%, more particularly greater than 60%, by weight of the encapsulating agent. The level of active agent retained may be determined by methods known in the art such as by hydro-distillation and solvent extraction in the case of flavor oils or by solvent extraction alone in the case of vitamins.

A high load level of active agent is desirable to reduce the cost of producing the final product as encapsulating agents are often expensive. Further, some encapsulating agents may contribute adverse or undesirable properties to the final system and it is thus desirable to reduce the amount of encapsulating agent used.

The load of the encapsulating agent may be limited in some instances by the processing of the end use form. For example, in a direct compression tablet, loading may be limited as high compression forces may result in leakage of the encapsulated oil, lowering the hardness of the resultant tablet and resistance to oxidation.

It is important not only to achieve a high load of active agent, but also to maintain it so as to enable a longer shelf life. Many active agents are volatile and/or labile, particularly flavors and fragrances. When the active agents are not encapsulated, they may be lost, producing undesirable variations in taste and aroma of the final products as perceived by the consumer. In addition, losses of such components increase the cost of the final products since it is necessary to increase the amount of the volatile/labile component to compensate for the losses which occur, and many are expensive.

In the case of oil as an active agent, the present encapsulating agents also retain the oil so as to provide a low surface oil. This is particularly true when glucoamylase is used to enzymatically hydrolyze the starch. The surface oil may be measured by methods known in the art such as by washing the encapsulated powder with a suitable solvent. Reduction of surface oil is important as increased surface oil indicates that the load of the active agent is not being maintained and inefficiency of encapsulation. Thus, reduction of surface oil results in a longer shelf life.

The present encapsulating agents also provide a relatively high level of oxidation resistance, thereby prolonging storage stability of the encapsulated product and shelf life of the final product. Oxidation resistance may be measured by methods known in the art. For example, oxidation resistance of encapsulating agents containing citrus oil may be determined by using gas chromatography (GC) to measure the amount of oxidization products of limonene, such as carvone, carviol, or limonene oxide, present in the oil extracted from powders aged at 50°C for two weeks: less than about 0.8% carvone typically indicates acceptable levels of oxidation. Oxidation resistance is important not only for flavor considerations of the oil, but also to maintain the activity of various vitamins. To further increase oxidation resistance, an anti-oxidant may be added to the oil.

The encapsulated product is effective when stored as a powder and spontaneously releases the active agent upon exposure to moisture. The resultant encapsulated product may be used at any level desired in food products, the amount being dependent upon the amount of active agent to be incorporated. In general, the starch will be used in an amount of from about 0.01 to about 10%, particularly from about 0.1 to about 5% by weight of the food product.

The resultant starch can be used in various food products including, but not limited to, cereals; powdered drink mixes; instant coffees and teas; powdered sauce and gravy mixes; instant soups; cereals; powdered dressings; bakery products; flavors; fragrances; colorants; and other dry food products. Upon preparation of these powdered and instant products, the moisture triggers the release mechanism, providing the active agent to the consumer.

The resultant starch may also be used in a variety of pharmaceuticals including vitamins; personal care products including antiperspirants, deodorants, soaps, fragrances, and cosmetics; hair care products, such as hair sprays, mousses, shampoos, cream rinses, and gels; paper products such as diapers, sanitary napkins, paper towels, tissues, toilet tissues; animal care products such as kitty litter; and household products such as carpet cleaners, and air fresheners.

The encapsulated product may also be used in a solid tablet-like form for a variety of applications, including detergents, foods and beverages, bath oils, agricultural products, and pharmaceuticals. The encapsulated products are particularly suitable for pharmaceutical tablets, including effervescent tablets.

The encapsulated product may generally be used at the desired level, the amount being dependent upon the amount of active agent to be incorporated, the desired hardness of the tablet, and the oxidative resistance desired. In general, the encapsulated product will be used in an amount of from about 1 to about 95% by weight of the tablet allowing for the active agent to be incorporated in an amount of from about 1 to about 60, particularly from about 10 to about 50%, by weight of the tablet.

The encapsulated product is particularly useful in a compressed tablet. The compressed tablet may be made using any method known in the art, particularly by direct compression of the tablet components. In the alternative, the tablet may be prepared by dry blending the encapsulated product with the other components of the formulation, granulating the mixture such as by fluid bed technology, roller compactor, extrusion, or high shear granulator, and dry compacting to a tablet.

Pharmaceutical excipients known in the art may be added to the pharmaceutical dosage form to impart satisfactory processing, compression, and disintegration characteristics to the formulation. Such excipients include, but are not limited to, diluents, flow enhancer, binders, lubricants and glidants, disintegrants, colors, flavors and sweetening agents. These excipients are well known in the art and are limited only by compatibility and characteristics desired.

Binders for the present invention include gelatin, microcrystalline cellulose, sugars, carboxymethyl cellulose, methyl cellulose, polyvinyl pyrrolidone, acacia, alginic acid, guar gum, hydroxypropyl methylcellulose, polyethylene oxide and ethyl cellulose.

Lubricants and glidants include talc, magnesium stearate, calcium stearate, stearic acid, glyceryl behenate, mineral oil, polyethylene glycol, sodium stearyl fumarate, stearic acid, vegetable oil, zinc stearate, and silicon dioxide.

Disintegrants suitable for the present invention include starches, algins, gums, croscarmelose, crospovidone, sodium starch glycolate, sodium laurel sulfate, microcrystalline cellulose, polacrilin potassium, and methylcellulose.

Diluents suitable for the present invention include dicalcium phosphate, calcium sulfate, lactose, cellulose, Kaolin, mannitol, sodium chloride, starch, sugars, calcium carbonate, calcium phosphate, dextrates, dextrin, dextrose, fructose, sorbitol, sucrose, and microcrystalline cellulose.

In particular, a binder is added to the tablet formulation to provide a tablet with the desired hardness. In general the hardness of the resultant tablet is at least about 3, more particularly at least about 4, most particularly at least about 6 kilopascals (kP).

If the final desired product is other than a pharmaceutical dosage form, alternative additives known to those arts may be present. For example, flavors and fragrances in a bath oil tablet or surfactants in a detergent tablet.

Upon contact with water, the moisture triggers the release mechanism, allowing the active agent to be released from the encapsulating starch. For example, upon digestion of the pharmaceutical dosage forms, the active agent is released to the body.

The following examples are presented to further illustrate and explain the present invention and should not be taken as limiting in any regard. The following analytical tests were used to measure various parameters in the examples.

Determination of Dextrose Equivalents (DE)

The dextrose equivalent of starch may be determined by using the Reducing Sugars test described in Food Chemicals Codex, 4th ed., July 1, 1996. Section 5, General Tests and Assays, Appendix X: Carbohydrates (Starches, Sugars, and Related Substances) or Standard Analytical Method #E-26 for Dextrose Equivalent from the Corn Refiners Association.

Oxidation Resistance Analysis

Twenty (20) grams of the modified starch powder loosely filled, were placed in a one liter jar with a powder to air ratio of 1:25. The jar was capped tightly with teflon. The sample was placed in a 50°C oven for two weeks.

The oil was then distilled using hydro-distillation. The distilled oil was then analyzed using gas chromatography for fresh (limonene) and oxidized (carvone) components.

Surface Oil Analysis

The unencapsulated oil present on the surface of the particle is repeatedly extracted using organic solvents, such as pentane, to remove all the surface oil and the extracted oil is quantitatively determined using gas chromatography techniques known in the art.

Oil Retention (Loading) Analysis

To determine the oil retention of the encapsulated product, 15 grams of the spray dried, encapsulated oil and 150 ml distilled water are mixed to reconstituted the emulsion. The emulsion is heated to reflux and held for four hours. The mixture is then cooled and the separated oil is removed and weighed.

$$\% \text{Retention} = \frac{\text{volume of oil extracted X specific gravity of oil}}{\text{theoretical oil weight}} \text{ X } 100$$

Example 1 - Preparation of the Derivatized Starch

500 grams of waxy maize starch were slurried in 750 ml water. The pH was adjusted to 7.5 using 3% sodium hydroxide. 15 grams of octenylsuccinic anhydride (OSA) were added in one-third increments every thirty minutes while maintaining the pH at 7.5 using 3% sodium hydroxide and constant agitation. The starch was then filtered out and washed with 750 ml water. The starch was then reslurried in 500 ml water and the pH adjusted to 5.5 with 3:1 hydrochloric acid. The starch was then filtered, washed with 750 ml water, and air dried to produce an OSA starch.

Example 2 - Preparation of the Modified Starch

a. Using glucoamylase

100 grams of the OSA starch of Example 1 were slurried in 300 ml water and the pH adjusted to 5.5 using dilute hydrochloric acid. The slurry was gelatinized by jet cooking in a C1-339 jet cooker, commercially available from National Starch and Chemical Company, at 300°F, at a chamber pressure of 55 psi, and a slurry rate of 6ml/min with the steam valve open at 75° capacity.

The temperature of the starch solution was then decreased to 55°C. 0.05% glucoamylase (AMG 200 L, commercially available from Novo Nordisk) based on the weight of the starch was added and the reaction was allowed to proceed at 55°C with constant mixing for approximately 2.5 hours until a dextrose equivalent of 36 and a viscosity of 17 sec at 25% solids and 22°C using the funnel method. The enzyme was then deactivated by heating the dispersion to 90°C and maintaining the elevated temperature for 30 minutes. The dispersion was then cooled to room temperature and spray dried using an inlet temperature of 200°C, an outlet temperature of 100°C and a feed rate of 65 ml/min.

b. Using β-amylase

100 grams of the OSA starch of Example 1 were slurried in 300 ml water and the pH adjusted to 5.5 using dilute hydrochloric acid. The slurry was gelatinized by jet cooking in a C1-339 jet cooker, commercially available from National Starch and Chemical Company, at 300°F, at a chamber pressure of 55 psi, and a slurry rate of 6ml/min with the steam valve open at 75% capacity.

The temperature of the starch solution was then decreased to 55°C. 0.2% β-amylase (Spezyme BBA 1500, commercially available from Genencor) based on the weight of the starch was added and the reaction was allowed to proceed at 55°C with constant mixing for approximately 4 hours until a dextrose equivalent of 36 and a viscosity of 17 sec at 25% solids and 22°C using the funnel method. The enzyme was then deactivated by heating the dispersion to 90°C and maintaining the elevated temperature for 30 minutes. The dispersion was then cooled to room temperature and spray dried using an inlet temperature of 200°C, an outlet temperature of 100°C and a feed rate of 65 ml/min.

c. Using a Combination of β-amylase and Pullulanase

100 grams of the OSA starch of Example 1 were slurried in 300 ml water and the pH adjusted to 5.25 using dilute hydrochloric acid. The slurry was gelatinized by jet cooking in a C1-339 jet cooker, commercially available from National Starch and Chemical Company, at 290°F, at a chamber pressure of 40 psi, and a slurry rate of 3.5ml/min with the steam valve open at 75% capacity.

The temperature of the starch solution was then decreased to 58°C. 5.0% of pullulanase (Promozyme, commercially available from Novo) by weight of starch was added and allowed to react for approximately 18 hours with constant mixing. Then 0.1% β-amylase (Spezyme BBA 1500, commercially available from Genencor) based on the weight of the starch was added and the reaction was allowed to proceed at 58°C with constant mixing for approximately 2.5 hours until a dextrose equivalent of 32 and a viscosity of 14 sec at 25% solids and 22°C using the funnel method. The enzymes were then deactivated by heating the dispersion to 95°C and maintaining the elevated temperature for 30 minutes. The dispersion was then cooled to room temperature and spray dried using an inlet temperature of 200°C, an outlet temperature of 100°C and a feed rate of 65 ml/min.

Example 3 - Encapsulation of Orange Oil with 40% Load

240 grams of the modified starch prepared in Example 2b was dispersed in 600 ml water in a high dispersion mill. The temperature was raised to 60°C until the starch dissolution appeared complete and then was lowered to 40°C. 160g of a single pressed orange oil commercially available from Givaudan-Roure was added and emulsified at high speed for approximately three minutes. The viscosity of the emulsion as determined using a Brookfield Viscometer Model 1+ using a small sample adaptor with spindle #18 is 85 cps at 40°C. The emulsion was spray dried to a powder.

The resultant encapsulated orange oil retained 38% oil based on the weight of the product, a 95% encapsulation of the oil used in the system; the surface oil (extractable oil) was 0.3%; the oxidation was found to be at acceptable levels after aging, 0.8% Carvone; and the moisture of the product was 1.9% as determined by the Karl-Fischer Method.

Example 4 - Preparation of an Orange Drink Mix

| Ingredient | Amount (grams) |
|---|---|
| Example 3 encapsulated oil | 2.18 |
| Sugar | 95.58 |
| Citric Acid | 1.74 |
| FD&C Yellow #15 | 0.03 |
| FD&C Yellow #6 | 0.03 |
| Benzoic Acid | 0.44 |

The ingredients were dry blended to prepare a powdered orange drink mix. 11.51 grams of the mix were reconstituted with 88.5 ml water to produce a clean-tasting orange drink which was free from oxidized flavors.

Example 5 - Encapsulation of Vitamin E

a. 165 grams of the starch of example 2b were dispersed in 670 grams water in a high dispersion mill. The temperature was raised to 60°C until the starch dissolution appeared complete and then was lowered to 40°C. 165 g of Vitamin E was added and emulsified at high speed for approximately three minutes. The emulsion was spray dried to a powder which contained 50% of 1000 IU Vitamin E.
b. Example 5a was repeated using the starch of example 2a.
c. Example 5a was repeated using a zero bloom fish gelatin.
d. Example 5a was repeated using CAPSUL® starch, an encapsulating starch commercially available from National Starch and Chemical Company in Bridgewater, New Jersey.

Example 6 - Use of the Modified Starch in a Vitamin E Tablet

The encapsulated vitamin E of Example 5a was made into compressed tablets.

| Ingredient | Amount (mg) |
|---|---|
| Example 5a encapsulated vitamin E | 4000.0 |
| Magnesium Stearate[1] | 25.0 |
| Amorphous fumed silica[2] | 25.0 |
| Microcrystalline cellulose[3] | 950.0 |
| [1] Magnesium stearate is commercially available from Witco. [2] Amorphous fumed silica is commercially available under the tradename Cab-O-Sil M5 P from Cabot. [3] Microcrystalline cellulose is commercially available under the tradename Avicel PH102 from FMC. | |

The ingredients were dry blended, 500 mg portions were weighed out and loaded into the press to form tablets using a Riva Piccola 10-station lab scale press at 600, 1500, and 5900 pounds force.
The hardness of these tablets were tested using a Pharmatron Model 6d Tablet Tester and disintegration was tested using an Erweka ZT71 Disintegration Tester. The results are listed below in Table I.

**EP 0 922 449 B1**

Table 1

| Compression Force (lbs). | Hardness (kP) | Disintegration (hh:mm:ss) |
|---|---|---|
| | | |
| 600 | 4.5 | 0:24:33 |
| 1500 | 4.0 | 1:36:01 |
| 5900 | 3.1 | 1:04:16 |

As can be seen from Table I, the encapsulating starch of the present invention provides good compressibility and sustable tablet hardness.

Example 7 - Use of the Modified Starch in a Vitamin E Tablet

Example 6 was repeated using the following formulation:

| Ingredient | Amount (mg) |
|---|---|
| Encapsulated Vitamin E | 4450.0 |
| Magnesium Stearate[1] | 25.0 |
| Amorphous fumed silica[2] | 25.0 |
| Microcrystalline cellulose[3] | 500.0 |

a. The encapsulated Vitamin E of example 5a was used. The results are listed below in Table II.
b. The encapsulated Vitamin E of example 5b was used. The results are listed below in Table II.
c. The encapsulated Vitamin E of example 5c was used. The results are listed below in Table II.
d. The encapsulated Vitamin E of example 5d was used. The results are listed below in Table II.

Table II

| Example | Compression Force (lbs). | Hardness (kP) | Disintegration (hh:mm:ss) |
|---|---|---|---|
| 7a | 573 | 5.6 | 0:49:16 |
| | 1695 | 6.1 | 0:40:20 |
| | 4691 | 5.6 | 0:35:24 |
| 7b | 494 | 3.3 | 0:23:01 |
| | 1835 | 3.7 | 0:16:43 |
| | 4466 | 3.6 | 0:16:47 |
| 7c | 773 | 4.7 | 0:25:59 |
| | 1770 | 4.6 | 0:24:25 |
| | 4417 | 5.8 | 0:22:01 |
| 7d | 467 | 1.5 | 0:10:38 |
| | 877 | 1.3 | 0:16:04 |
| | 4156 | 1.1 | 0:09:34 |

As can be seen from Table II, the encapsulating starches of the present invention provide good compressibility and suitable tablet hardness. In many cases, the present invention was at least comparable to the standard gelatin. Further, different hardnesses may be achieved by varying the formulation and the compression force.

Example 8 - Use of the Modified Starch in a Vitamin E Tablet

Example 6 was repeated using the following formulation:

| Ingredient | Amount (mg) |
|---|---|
| Encapsulated Vitamin E | 4700.0 |
| Magnesium Stearate[1] | 25.0 |
| Amorphous fumed silica[2] | 25.0 |
| Microcrystalline cellulose[3] | 250.0 |

a. The encapsulated Vitamin E of example 5a was used. The results are listed below in Table III.
b. The encapsulated Vitamin E of example 5b was used. The results are listed below in Table III.
c. The encapsulated Vitamin E of example 5c was used. The results are listed below in Table III.
d. The encapsulated Vitamin E of example 5d was used. The results are listed below in Table III.

Table III

| Example | Compression Force (lbs). | Hardness (kP) | Disintegration (hh:mm:ss) |
|---|---|---|---|
| 8a | 477 | 3.0 | 0:29:35 |
| | 1293 | 3.26 | 0:35:38 |
| | 5089 | 3.12 | 0:26:13 |
| 8b | 566 | 1.4 | 0:11:29 |
| | 1386 | 1.68 | 0:13:33 |
| | 5322 | 1.28 | 0:11:46 |
| 8c | 565 | 1.34 | 0:23:21 |
| | 1223 | 1.92 | 0:24:39 |
| | 5543 | 2.16 | 0:18:46 |
| 8d | 504 | 0.32 | 0:11:53 |
| | 1627 | 0.46 | 0:13:55 |
| | 5326 | 0.18 | 0:15:28 |

As can be seen from Table III, the encapsulating starches of the present invention provide good compressibility and suitable tablet hardness. In many cases, the present invention was at least comparable to the standard gelatin. Further, different hardnesses may be achieved by varying the formulation and the compression force.

Example 9 - Preparation of an Orange Oil Tablet and Its Oxidation Resistance

500 mg portions of the encapsulated orange oil of Example 3 were weighed out and loaded into the press to form tablets using a Riva Piccola 10-station lab scale press at 2500 pounds force.

The oxidation resistance of the tablet was tested by re-emulsifying 100 mg of the tablet in 2 ml water. The samples were analyzed using static headspace - GC/FID to determine the level of components indicative of limonene (orange oil) oxidation. The results are shown in Table IV below.

| Tablet | % limonene epoxide | % carvone | % carveol |
|---|---|---|---|
| 1 | 0.26 | 0 36 | 0.40 |
| 2 | 0.29 | 030 | 0.41 |

As can be seen from Table IV, the oxidation of the tabletted orange oil was low, showing that the encapsulating starch provides excellent oxidation resistance in a tablet.

Example 10 - Preparation of a Derivatized Starch/Sugar Mixture

a. 600 grams of an OSA derivatized starch prepared as in Example 1 were mixed with 400 grams glucose.
b. A cold water soluble, mildly acid degraded OSA starch was prepared by using the starch of Example 1, hydrolyzing using acid to a fluidity of about 60, and spray drying the starch. 600 grams of this starch were mixed with 400 grams glucose.
c. 500 grams of the starch prepared as in Example 10b were mixed with 500 grams maltose.
d. 340 grams of an OSA derivatized starch prepared as in Example 1 were mixed with 660 grams maltose.
e. 550 grams of an OSA derivatized starch prepared as in Example 1 were mixed with 450 grams glucose.
f. 500 grams of an OSA derivatized starch prepared as in Example 1 were mixed with 500 grams glucose.
g. 400 grams of an OSA derivatized starch prepared as in Example 1 were mixed with 600 grams glucose.

Example 11 - Analysis of Encapsulation

The modified starches and starch/sugar blends were used to encapsulate orange oil as in Example 3 with the following conditions/parameters.

| Encap. Example | Starch | Emulsion Properties | | | Spray Dryer Conditions | | |
|---|---|---|---|---|---|---|---|
| | | %Solids | Emulsion Viscosity (cps) | Particle Size ($\mu$) | Feed Rate (ml/min) | Inlet Temp. (°C) | Outlet Temp. (°C) |
| 11a | Example 10a | 37.0 | 357 | 1.0 | 150.0 | 195 | 103 |
| 11b | Example 10b | 35.0 | 400 | 0.9 | 175 | 191 | 105 |
| 11c | Example 10c | 40.0 | 357 | 1.0 | 175 | 193 | 106 |
| 11d | Example 10d | 29.0 | 110 | 1.3 | 150 | 194 | 104 |
| 11e | Example 10e | 22.0 | 155 | 1.1 | 150 | 193 | 115 |
| 11f | Example 10f | 33.0 | 460 | 1.2 | 150 | 185 | 90 |
| 11g | Example 10e | 35.8 | 400 | 1.0 | 175 | 185 | 92 |
| 11h | Example 10g | 37.0 | 425 | 1.4 | 150 | 185 | 98 |

The results of the encapsulation are listed below.

| | Time = 0 | | Time = 2 weeks at 50°C | |
|---|---|---|---|---|
| | % Retention | % Surface Oil | % Retention | % Carvone |
| Example 11a | 97.0 | 0.1 | 96.0 | 0.50 |
| Example 11b | 98.5 | 0.3 | 95.7 | 0.40 |
| Example 11c | 94.7 | 1.2 | 93.3 | 0.30 |

(continued)

| | Time = 0 | | Time = 2 weeks at 50°C | |
|---|---|---|---|---|
| | % Retention | % Surface Oil | % Retention | % Carvone |
| Example 11d | 83.0 | 1.9 | 80.0 | 0.50 |
| Example 11e | 91.0 | 0.3 | 83.0 | 1.00 |
| Example 11 f | 96.0 | 0.1 | 92.0 | 0.70 |
| Example 11g | 95.0 | 0.1 | 92.0 | 0.80 |
| Example 11 h | 96.0 | 0.1 | 94.5 | 0.40 |

Example 12 - Analysis of Encapsulation

A variety of glucoamylase, β-amylase, and pullulanase/β-amylase OSA starches were prepared using the method of Example 2 with the exception that hydrolysis was allowed to proceed to different degrees. The modified starches were used to encapsulate orange oil as in Example 3. The results of the encapsulation are reported below.

| Enzyme | DE | Viscosity at 22°C (sec) | | %Retention | | %Surface Oil | %Carvone |
|---|---|---|---|---|---|---|---|
| | | 19% solids | 25% solids | Time=0 | Time = 2 weeks at 50°C | | |
| β-amylase* | 19.8 | --- | --- | 68.42 | 50.00 | 13.71 | 3.55 |
| β-amylase | 32.0 | 18.6 | --- | 84.21 | 84.21 | 1.55 | 0.83 |
| β-amylase | 32.8 | 9.5 | 14.9 | 91.86 | 87.08 | 1.33 | 0.33 |
| β-amylase | 32.8 | 8.2 | 11.2 | 89.89 | 84.27 | 1.59 | 0.64 |
| β-amylase | 36.0 | 7.9 | 10.1 | 96.05 | 94.63 | 0.22 | 0.33 |
| β-amylase | 34 | 15.4 | 18.5 | 97.77 | 93.58 | 0.40 | 0.47 |
| β-amylase | 32 | --- | --- | 89.20 | 81.25 | 1.90 | 0.85 |
| β-amylase | 32 | --- | --- | 91.90 | 87.70 | 1.33 | 0.38 |
| pullulanase/ β-amylase | 32.2 | 9.6 | 13.9 | 94.14 | 98.92 | 1.05 | 0.18 |
| Glucoamylase* | 19.7 | --- | --- | 84.21 | 73.68 | 3.87 | 2.10 |
| Glucoamylase | 30.3 | 10.7 | 19.8 | 86.61 | 78.95 | 1.31 | 1.12 |
| Glucoamylase | 37.7 | 9.7 | 15.3 | 98.31 | 95.51 | 0.08 | 0.36 |
| Glucoamylase | 48.3 | 7.7 | 9.5 | 91.62 | 89.47 | 0.19 | 0.58 |
| Glucoamylase | 48.3 | 7.7 | 9.5 | 91.62 | 89.24 | 0.05 | .029 |
| Glucoamylase | 30 | --- | --- | 98.20 | 81.24 | 1.94 | 1.06 |
| Glucoamylase | 45 | --- | --- | 91.60 | 91.20 | 0.05 | 0.52 |
| *not forming part of the invention | | | | | | | |

Example 13 - Preparation and Analysis of 50% Load Orange Oil

110 grams of the derivatized starch of Example 1 and 90 grams glucose were blended and dispersed in 600 ml water in a high dispersion mill. The temperature was raised to 60°C until the starch dissolution appeared complete and then was lowered to 40°C. 200 g of a single pressed orange oil commercially available from Givaudan-Roure was added and emulsified at high speed for approximately three minutes. The viscosity of the emulsion as determined using a Brookfield Viscometer Model 1 + using a small sample adaptor with spindle #18 is 425 cps at 40°C. The emulsion was spray dried to a fine powder.

The resultant encapsulated orange oil retained 38% oil based on the weight of the product, a 95% encapsulation of the oil used in the system; the surface oil (extractable oil) was 0.3%, the oxidation was found to be at acceptable levels after aging, 0.8% Carvone; and the moisture of the product was 1.9% as determined by the Karl-Fischer Method.

Example 14 - Comparison with other encapsulating starches

Starch example 14a is a 50:50 blend of corn syrup solids and cold water soluble, acid degraded, OSA starch in which the starch is prepared by acid hydrolyzing the OSA starch of Example 1 to a water fluidity of about 65 and then spray drying.
Example 14b is the OSA starch of example 1.
The modified starches were used to encapsulate orange oil as in Example 3. The results of the encapsulation as reported below.

| Example | DE | %Retention | | %Surface Oil | %Carvone |
|---------|-----|----------|-------------------------|-------------|----------|
| | | Time=0 | Time = 2 weeks at 50°C | | |
| 14a | 23 | 84.20 | 74.40 | 4.54 | 1.03 |
| 14b | 0 | 56.0 | --- | 12.0 | --- |

No stability test was run on example 11b due to the poor retention. A comparison of the above results with those of examples 8 and 9 shows the superior retention, and decreased surface oil and oxidation of the present starches.

Example 15 - Preparation of an Antiperspirant

| Ingredient | Amount (grams) |
|------------|----------------|
| Encapsulated fragrance | 1.00 |
| Dow Corning Fluid 344 | 49.0 |
| Cyclochem EDGS | 1.00 |
| Arlacel 165 | 1.00 |
| Promyristyl PM3 | 5.00 |
| Crodacol S-95NF | 17.00 |
| Rezal 36 GP Suf | 20.00 |
| DRY-FLO® PC Starch | 6.00 |

The encapsulated fragrance is prepared by the methodology of Example 3, substituting the fragrance for the orange oil.

Dow Corning Fluid 344 is cyclomethicone commercially available from Dow Corning.
Cyclochem EDGS is glycol distearate commercially available from Alcolac.
Arlacel 165 is glyceryl stearate and PEG 100 stearate commercially available from ICI.
Promyristyl PM3 is PPG-3 myristyl ether commercially available from Croda.
Crodacol S-95NF is stearyl alcohol commercially available from Croda.
Rezal 36 GP Suf is aluminum zirconium tetrachlorohydrex glycine commercially available from Reheis.
DRY-FLO® PC starch, a modified food starch used as a dusting and lubricating agent, is commercially available from National Starch and Chemical Company.

The Dow Corning Fluid 344, Cyclochem EDGS, Arlacel 165, and Promyristyl PM3 are mixed and heated to 65°C. The Crodacol S-95NF is mixed in thoroughly. The Rezal 36 GP Suf is added and mixed for ten minutes. The DRY-FLO® starch is added and mixed thoroughly. Then the encapsulated fragrance is added and mixed thoroughly. The mixture is cooled to 50°C, poured into molds, and cooled to room temperature.

14

Example 16 - Preparation of a Detergent

| Ingredient | Amount (g) |
|---|---|
| Sodium lauryl sulfate | 15 |
| Sodium lauryl ether sulphate | 10 |
| Magnesium aluminum silicate | 30 |
| Sodium Carbonate | 19.5 |
| Sodium disilicate | 2 |
| Sodium Perborate | 15 |
| Sodium polycarboxylate (acrylic/maleic) | 3 |
| Polyester terephthalate | 2 |
| Protease enzyme | 0.5 |
| Encapsulated fragrance | 0.75 |
| Optical brightener | 0.2 |
| Sodium phosphonate | 1.55 |
| TAED bleach activator | 0.5 |

The fragrance is encapsulated using the starch of Example 2b and the method of Example 3 in which the fragrance is substituted for the orange oil. The ingredients are blended together.

Example 17 - Preparation of a Phosphate-Free Detergent

| Ingredient | Amount (g) |
|---|---|
| Magnesium Aluminum Silicate | 30 |
| Sodium Percarbonate | 15 |
| Sodium Alkyl Sulphate | 15 |
| Sodium Alcohol Ethoxylate | 10 |
| Dimethylamine Oxide | 6.75 |
| Sodium Carbonate | 15 |
| Sodium Sulphate | 7 |
| Encapsulated Fragrance | 1.25 |

The fragrance is encapsulated using the starch of Example 2b and the method of Example 3 in which the fragrance is substituted for the orange oil The ingredients are blended together.

Claims

1. An encapsulating agent comprising a modified starch comprising a starch derivative containing a hydrophobic group or both a hydrophobic and a hydrophilic group which has been degraded by at least one enzyme capable of cleaving the 1,4-linkages of the starch molecule from the non-reducing ends to produce short chained saccharides to provide high oxidation resistance while maintaining substantially high molecular weight portions of the starch base to provide encapsulating properties, wherein the starch is degraded to a dextrose equivalent of from 20 and up to 50.

**2.** The encapsulating agent of claim 1, wherein the enzyme is selected from the group consisting of β-amylase, glucoamylase, pullulanase, maltogenase, exo-alpha-1,4-glucosidase, exo-1,4-alpha-D-glucan maltotetrahydrolase, and exo-1,4-alpha-D glucan maltohexahydrolase.

**3.** The encapsulating agent of claim 2, wherein the enzyme is β-amylase or glucoamylase.

**4.** The encapsulating agent of claim 3, wherein the enzyme is β-amylase.

**5.** The encapsulating agent of claim 4, wherein the starch is degraded to a dextrose equivalent of from 25 to 38.

**6.** The encapsulating agent of any one of claims 1-8, wherein the starch has a viscosity of less than 30 seconds as measured by the funnel method.

**7.** The encapsulating agent of claim 6, wherein the starch has a viscosity of from 8 to 25 seconds.

**8.** The encapsulating agent of claim 7, wherein the starch has a viscosity of from 8 to 15 seconds.

**9.** The encapsulating agent of any one of claims 1-8, wherein the starch derivative is gelatinized and the hydrophobic group comprises an alkyl, or an alkenyl group which contains at least five carbon atoms or an aralkyl or aralkenyl group which contains at least six carbon atoms.

**10.** The encapsulating agent of claim 9, wherein the starch is gelatinized and has been derivatized by treatment with at least 0.1% of octenyl succinic acid anhydride on a starch dry weight basis.

**11.** A method of encapsulating an active agent comprising the encapsulating agent of claim 1, the method comprising:

    a) forming a solution of the encapsulating agent; and
    b) emulsifying the active agent in the solution.

**12.** The method of claim 11, further comprising drying the emulsion to remove the water therefrom.

**13.** The method of claim 12 wherein the drying step is accomplished by spray drying.

**14.** An encapsulated active agent comprising an active agent and the encapsulating agent of any one of claims 10.

**15.** The encapsulated active agent of claim 14. wherein the active agent is selected from the group consisting of flavors, colors, fragrances, vitamins, and pharmaceuticals.

**16.** The encapsulated active agent of claim 14 or 15 having a load of a least 40%, low surface oil, and high oxidation resistance.

**17.** The encapsulated active agent of any one of claims 14-16 having a load of a least 50%, low surface oil, and high oxidation resistance.

**18.** The encapsulated active agent of claim 17 having a load of a least 60%, low surface oil, and high oxidation resistance.

**19.** A food composition comprising the encapsulated active agent of any one of claims 14-18.

**20.** A composition comprising the encapsulated active agent of any one of claims 14-18, the composition being selected from the group consisting of a pharmaceutical, a personal care product, a hair care product, a paper product, an animal care product, and a household product.

**21.** A tablet comprising

    a) an encapsulating agent comprising the starch of any one of claims 1-10; and
    b) an active agent.

**22.** The tablet of claim 21, wherein the active agent is selected from the group consisting of a vitamin, a pharmaceutical, a pesticide, an oil, a protein, a fat, a flavor, a color, a catalyst, and a fragrance.

23. The tablet of claim 21 or 22, wherein the active agent is selected from the group consisting of a botanical oil, an essential oil; Vitamin A, Vitamin D, Vitamin E, Vitamin K; a peptide, an amino acid, an enzyme; and an oil soluble pharmaceutical, oil soluble pesticide.

24. The tablet of claim 23, wherein the active agent is Vitamin E.

25. The tablet of any one of claims 21-24, wherein the tablet has a hardness of at least 3 kilopascals.

26. The tablet of claim 25, wherein the tablet has a hardness of at least 4 kilopascals.

**Patentansprüche**

1. Einkapselungsmaterial, umfassend eine modifizierte Stärke, die ein Stärkederivat umfasst, das eine hydrophobe Gruppe oder sowohl eine hydrophobe als auch eine hydrophile Gruppe umfasst, die durch wenigstens ein Enzym abgebaut wurde, das fähig ist, die 1,4-Verknüpfungen des Stärkemoleküls von den nicht-reduzierenden Enden aus unter Herstellung kurzkettiger Saccharide zu spalten, um hohe Oxidationsbeständigkeit bereit zu stellen, während Teile der Stärkegrundlage mit hohem Molekulargewicht im Wesentlichen aufrecht erhalten bleiben, um Einkapselungseigenschaften bereit zu stellen, wobei die Stärke bis zu einem Dextroseäquivalent von 20 und bis zu 50 abgebaut ist.

2. Einkapselungsmaterial nach Anspruch 1, wobei das Enzym aus der Gruppe, bestehend aus ß-Amylase, Glucoamylase, Pullulanase, Maltogenase, Exo-alpha-1,4-glucosidase, Exo-1,4-alpha-D-glucan-maltotetrahydrolase und Exo-1,4-alpha-D-glucan-maltahexhydrolase, ausgewählt ist.

3. Einkapselungsmaterial nach Anspruch 2, wobei das Enzym ß-Amylase oder Glucoamylase ist.

4. Einkapselungsmaterial nach Anspruch 3, wobei das Enzym ß-Amylase ist.

5. Einkapselungsmaterial nach Anspruch 4, wobei die Stärke zu einem Dextroseäquivalent von 25 bis 38 abgebaut ist.

6. Einkapselungsmaterial nach einem der Ansprüche 1 bis 5, wobei die Stärke eine Viskosität, gemessen durch das Trichterverfahren, von weniger als 30 Sekunden hat.

7. Einkapselungsmaterial nach Anspruch 6, wobei die Stärke eine Viskosität von 8 bis 25 Sekunden hat.

8. Einkapselungsmaterial nach Anspruch 7, wobei die Stärke eine Viskosität von 8 bis 15 Sekunden hat.

9. Einkapselungsmaterial nach einem der Ansprüche 1 bis 8, wobei das Stärkederivat gelatiniert ist und die hydrophobe Gruppe eine Alkyl- oder eine Alkenyl-Gruppe, die wenigstens fünf Kohlenstoffatome enthält, oder eine Aralkyl- oder Aralkenyl-Gruppe, die wenigstens sechs Kohlenstoffatome enthält, umfasst.

10. Einkapselungsmaterial nach Anspruch 9, wobei die Stärke gelatiniert ist und durch Behandlung mit wenigstens 0,1% Octenylbernsteinsäureanhydrid, bezogen auf das Stärketrockengewicht, derivatisiert wurde.

11. Verfahren zur Einkapselung eines aktiven Agenses, das das Einkapselungsmaterial nach Anspruch 1 umfasst, wobei das Verfahren umfasst:

   a) Bildung einer Lösung des Einkapselungsmaterials; und
   b) Emulgieren des aktiven Agenses in der Lösung.

12. Verfahren nach Anspruch 11, das außer dem Trocknen der Emulsion unter Entfernung des Wassers daraus umfasst.

13. Verfahren nach Anspruch 12, wobei der Trocknungsschritt durch Sprühtrocknung durchgeführt wird.

14. Eingekapseltes aktives Agens, das ein aktives Agens und das Einkapselungsmaterial nach einem der Ansprüche 1 bis 10 umfasst.

15. Eingekapseltes aktives Agens nach Anspruch 14, wobei das aktive Agens aus der Gruppe bestehend aus Aromastoffen, Färbemitteln, Riechstoffen, Vitaminen und Pharmazeutika, ausgewählt ist.

16. Eingekapseltes aktives Agens nach Anspruch 14 oder 15, das eine Beladung von mindestens 40%, geringes Oberflächenöl und hohe Oxidationsbeständigkeit hat.

17. Eingekapseltes aktives Agens nach einem der Ansprüche 14 bis 16, das eine Beladung von wenigstens 50%, geringes Oberflächenöl und hohe Oxidationsbeständigkeit hat.

18. Eingekapseltes aktives Agens nach Anspruch 17, das eine Beladung von wenigstens 60%, geringes Oberflächenöl und hohe Oxidationsbeständigkeit hat.

19. Nahrungsmittelzusammensetzung, die das eingekapselte aktive Agens nach einem der Ansprüche 14 bis 18 umfasst.

20. Zusammensetzung, die das eingekapselte aktive Agens nach einem der Ansprüche 14 bis 18 umfasst, wobei die Zusammensetzung aus der Gruppe, bestehend aus einem Pharmazeutikum, einem Körperpflegeprodukt, einem Haarpflegeprodukt, einem Papierprodukt, einem Tierpflegeprodukt und einem Haushaltsprodukt, ausgewählt ist.

21. Tablette, umfassend

a) ein Einkapselungsmaterial, das die Stärke nach einem der Ansprüche 1 bis 10 umfasst, und
b) ein aktives Agens.

22. Tablette nach Anspruch 21, wobei das aktive Agens aus der Gruppe, bestehend aus einem Vitamin, einem Pharmazeutikum, einem Pestizid, einem Öl, einem Protein, einem Fett, einem Aromamittel, einem Farbmittel, einem Katalysator und einem Riechstoff, ausgewählt ist.

23. Tablette nach Anspruch 21 oder 22, wobei das aktive Agens aus der Gruppe, bestehend aus einem botanischen Öl, einem ätherischen Öl; Vitamin A, Vitamin D, Vitamin E, Vitamin K; einem Peptid, einer Aminosäure, einem Enzym; und einem öllöslichen Pharmazeutikum, öllöslichen Pestizid, ausgewählt ist.

24. Tablette nach Anspruch 23, wobei das aktive Agens Vitamin E ist.

25. Tablette nach einem der Ansprüche 21 bis 24, wobei die Tablette eine Härte von wenigstens 3 Kilopascal hat.

26. Tablette nach Anspruch 25, wobei die Tablette eine Härte von 4 Kilopascal hat.


**Revendications**

1. Agent d'encapsulation comprenant un amidon modifié comprenant un dérivé d'amidon hydrophobe ou bien à la fois un groupe hydrophobe et un groupe hydrophile qui a été dégradé par au moins une enzyme capable de cliver les liaisons 1,4 de la molécule d'amidon à partir des extrémités non réductrices pour produire des saccharides à chaîne courte afin de conférer une grande résistance à l'oxydation tout en maintenant des portions de poids moléculaire substantiellement élevé de l'amidon de base afin de conférer des propriétés d'encapsulation, dans lequel l'amidon est dégradé à un équivalent de dextrose de 20 jusqu'à 50.

2. Agent d'encapsulation suivant la revendication 1, dans lequel l'enzyme est choisie dans le groupe consistant en la β-amylase, la glucoamylase, la pullulanase, la maltogénase, l'exo-alpha-1,4-glucosidase, l'exo-1,4-alpha-D-glucane-maltotétrahydrolase et l'exo-1,4-alpha-D-glucane-maltohexahydrolase.

3. Agent d'encapsulation suivant la revendication 2, dans lequel l'enzyme est la β-amylase ou la glucoamylase.

4. Agent d'encapsulation suivant la revendication 3, dans lequel l'enzyme est la β-amylase.

**5.** Agent d'encapsulation suivant la revendication 4, dans lequel l'amidon est dégradé à un équivalent de dextrose de 25 à 38.

**6.** Agent d'encapsulation suivant l'une quelconque des revendications 1 à 5, dans lequel l'amidon a une viscosité inférieure à 30 secondes, de la manière mesurée par la méthode à l'entonnoir.

**7.** Agent d'encapsulation suivant la revendication 6, dans lequel l'amidon a une viscosité de 8 à 25 secondes.

**8.** Agent d'encapsulation suivant la revendication 7, dans lequel l'amidon a une viscosité de 8 à 15 secondes.

**9.** Agent d'encapsulation suivant l'une quelconque des revendications 1 à 8, dans lequel le dérivé d'amidon est gélatinisé et le groupe hydrophobe comprend un groupe alkyle ou un groupe alcényle qui contient au moins cinq atomes de carbone ou un groupe aralkyle ou aralcényle qui contient au moins six atomes de carbone.

**10.** Agent d'encapsulation suivant la revendication 9, dans lequel l'amidon est gélatinisé et a été transformé en dérivé par traitement avec au moins 0,1 % d'anhydride d'acide octénylsuccinique sur la base du poids à sec de l'amidon.

**11.** Procédé pour encapsuler un agent actif comprenant l'agent d'encapsulation de la revendication 1, procédé comprenant :

a) la formation d'une solution de l'agent d'encapsulation ; et
b) l'émulsionnement de l'agent actif dans la solution.

**12.** Procédé suivant la revendication 11, comprenant en outre le séchage de l'émulsion pour en éliminer l'eau.

**13.** Procédé suivant la revendication 12, dans lequel l'étape de séchage est mise en oeuvre par séchage par pulvérisation.

**14.** Agent actif encapsulé comprenant un agent actif et l'agent d'encapsulation de l'une quelconque des revendications 1 à 10.

**15.** Agent actif encapsulé suivant la revendication 14, l'agent actif étant choisi dans le groupe consistant en des aromes, des colorants, des parfums, des vitamines et des agents pharmaceutiques.

**16.** Agent actif encapsulé suivant la revendication 14 ou 15, ayant une charge d'au moins 40 %, une faible quantité d'huile en surface et une grande résistance à l'oxydation.

**17.** Agent actif encapsulé suivant l'une quelconque des revendications 14 à 16, ayant une charge d'au moins 50 %, une faible quantité d'huile en surface et une grande résistance à l'oxydation.

**18.** Agent actif encapsulé suivant la revendication 17, ayant une charge d'au moins 60 %, une faible quantité d'huile en surface et une grande résistance à l'oxydation.

**19.** Composition alimentaire comprenant l'agent actif encapsulé de l'une quelconque des revendications 14 à 18.

**20.** Composition comprenant l'agent actif encapsulé de l'une quelconque des revendications 14 à 18, la composition étant choisie dans le groupe consistant en un agent pharmaceutique, un produit pour soins individuels, un produit pour soins capillaires, un produit en papier, un produit de soins pour animaux et un produit domestique.

**21.** Comprimé comprenant :

a) un agent d'encapsulation comprenant l'amidon de l'une quelconque des revendications 1 à 10 ; et
b) un agent actif.

**22.** Comprimé suivant la revendication 21, dans lequel l'agent actif est choisi dans le groupe consistant en une vitamine, un agent pharmaceutique, un pesticide, une huile, une protéine, une matière grasse, un arome, un colorant, un catalyseur et un parfum.

**23.** Comprimé suivant la revendication 21 ou 22, dans lequel l'agent actif est choisi dans le groupe consistant en une huile végétale, une huile essentielle, la vitamine A, la vitamine D, la vitamine E, la vitamine K ; un peptide, un aminoacide, une enzyme et un agent pharmaceutique soluble dans l'huile ou un pesticide soluble dans l'huile.

**24.** Comprimé suivant la revendication 23, dans lequel l'agent actif est la vitamine E.

**25.** Comprimé suivant l'une quelconque des revendications 21 à 24, le comprimé ayant une dureté d'au moins 3 kilopascals.

**26.** Comprimé suivant la revendication 25 le comprimé ayant une dureté d'au moins 4 kilopascals.